(19) Europäisches Patentamt
European Patent Office
Office européen des brevets

(11) EP 1 392 620 B1

(12) EUROPEAN PATENT SPECIFICATION

(45) Date of publication and mention of the grant of the patent:
**23.05.2007 Bulletin 2007/21**

(21) Application number: **02758223.8**

(22) Date of filing: **07.06.2002**

(51) Int Cl.:
***C07B 57/00*** *(2006.01)* ***C07C 307/02*** *(2006.01)*

(86) International application number:
**PCT/EP2002/006321**

(87) International publication number:
**WO 2002/100804 (19.12.2002 Gazette 2002/51)**

(54) **NOVEL USE OF SULFAMIC ACIDS AS RESOLVING AGENT.**

VERWENDUNG VON SULFAMIDSÄUREN ALS RACEMATSPALTUNGSMITTEL

NOUVELLE UTILISATION D'ACIDES SULFAMIQUES COMME AGENTS DE SEPARATION

(84) Designated Contracting States:
**AT BE CH CY DE DK ES FI FR GB GR IE IT LI LU MC NL PT SE TR**

(30) Priority: **08.06.2001 EP 01202251**
**08.06.2001 US 296484 P**

(43) Date of publication of application:
**03.03.2004 Bulletin 2004/10**

(73) Proprietor: **Avantium International B.V.**
**1014 BV Amsterdam (NL)**

(72) Inventors:
- **SMITH, Alan Arthur**
**2106 El Heemstede (NL)**
- **DAMEN, Franciscus Wilhelmus Petrus**
**6538 TE Nijmegen (NL)**
- **KUSTER, George Johannes Theodorus**
**NL-6541 ZX Nijmegen (NL)**

(74) Representative: **Wittop Koning, Tom Hugo et al**
**Exter Polak & Charlouis B.V.,**
**P.O. Box 3241**
**2280 GE Rijswijk (NL)**

(56) References cited:
**US-A- 4 582 928**

- **K.B. WIBERG: "Organic Syntheses, vol. 49" 1969 , JOHN WILEY AND SONS , NEW YORK, NY, US XP002182283 page 93 -page 98**
- **A.H. BLATT: "Organic Syntheses, Collective Volume 2" 1946 , JOHN WILEY AND SONS , NEW YORK, NY, US XP002182284 page 506 -page 509**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 392 620 B1

## Description

The invention relates to the use of chiral sulfamic acids as resolving agent for resolving an enantiomeric mixture, in particular a mixture of basic enantiomers, and to methods for resolving such enantiomeric mixtures.

The term "enantiomeric mixture" is known in the art and is understood to mean a mixture of two enantiomers.

The term "resolving enantiomers" is understood in the art to mean the increase of the relative amount of a particular enantiomer in an enantiomeric mixture. Such a resolving method can be used to obtain an enantiomerically pure compound from an enantiomeric mixture. The term "resolving agent" used herein is understood to mean an agent that can be used for resolving enantiomers.

Resolving methods, known in the art are based on preferential crystallisation of diastereomeric salts, formed by a reaction of the resolving agent with the enantiomeric compounds in the mixture. Diastereomeric salts are known to have different physical properties, such as crystallisation characteristics; it is known in the art to separate diastereomeric salts from one another based on the said different characteristics, by choosing the proper conditions therefor. Ideally, only the salt of one enantiomeric form of the enantiomeric compound of the mixture precipitates under the chosen conditions, whereas the salts of the other enantiomer remain in solution. Said precipitate can be further purified, resulting in an enantiomerically enriched, or enantiomerically pure compound. Instead of, or in addition to the precipitated fraction, the liquid fraction, the so-called "mother liquor" can be used for the purification of the non-precipitated salt, comprising the antipodic enantiomer of the mixture in enantiomerically enriched form. Herein, an enantiomer is deemed to be "enantiomerically enriched" when the said enantiomer is present in a higher molar amount than the other (antipodic) enantiomer.

Resolution of racemic compounds through formation and separation of diastereomeric salts is an important technology for the preparation of enantiopure products on an industrial scale. The finding of a suitable resolving agent is however, often, trial and error.

In the art, carboxylic acids are known as resolving agents for resolving a mixture of basic enantiomers; however, such compounds have a relatively weak acidity, resulting in limiting salt formation, which is particularly problematic for resolving weak basic compounds.

Further, 10-camphorsulfonic acid is known as resolving agent for basic enantiomeric mixtures, see Stereochem. Org. Compounds, E.L. Eliel, S.H. Wilen, Wiley Interscience, N.Y., U.S.A., 1994, pp. 322-337. However, the diversity in resolving agents, in particular in resolving weak basic enantiomers, in limited.

According to the invention, it is now found that chiral sulfamic acids having an enantiomeric purity of at least 90% of formula (I):

$$(R_1, R_2)N\text{-}SO_3H \qquad (I)$$

wherein:

$R_1$ and $R_2$, being the same or different, are selected from the group consisting of:

- a hydrogen atom,
- a linear, branched or cyclic alkyl or heteroalkyl group, being optionally substituted, or
- an aromatic or heteroaromatic group, being optionally substituted, provided that $R_1$ and $R_2$ are not both hydrogen and that at least one of the $R_1$ and $R_2$ groups is chiral,

have optimal and surprising properties rendering these compounds highly suitable for use as resolving agent for resolving a mixture of enantiomers, resulting in efficient resolution of mixtures of enantiomeric compounds.

The said sulfamic acids have a stronger acidity than corresponding carboxylic acids and will therefore have an increased potency for salt formation, particularly with weakly basic compounds. Thus the current method provides a significant enhancement in diversity.

The term "enantiomeric purity of at least 90%" means that, on a molar basis, at least 95% of the sulfamic acid has the same stereochemic conformation; this means that less than 5% of the sulfamic acid is of the antipodic stereochemic conformation. This can be derived from the known formula for enantiomeric purity

$$[(A-B)/(A+B)] \times 100\%,$$

wherein A and B stand for each of the enantiomers respectively. It is to be noted that the maximum obtainable resolution of the enantiomers in the mixture corresponds to the enantiomeric purity of the resolving agent; this means that when the resolving agent has an enantiomeric purity of 95%, the maximum obtainable resolution of the enantiomers will be 95%.

Sulfamic acids as such and the preparation thereof from amines are known in the art; US 2 933 513 describes the preparation of (salts of) testosterone sulfamic acids. These compounds are used as water-soluble analogues of testosterone. A.G. Lloyd, et a1. (Biochemical Journal 92, 1964, 68-72) discloses the synthesis of labeled sulfamic acids of D-glucosamine for metabolism studies. S.-K. Chung, *et al.* (Tetrahedron 54, 1998, 15899-15914) describe the synthesis of sulfamic acid steroid derivatives for use as antifungal agents. M.L. Wolfrom, *et al.* (Journal of the American Chemical Society 79, 1957, 5043-5046) disclose the synthesis of D-glucose derivatives containing a sulfamic acid group by reaction of D-glucosamine free base with $SO_3$-pyridine complex. This unit is present in heparin. M.H. Payne, *et al.* (Journal of Medicinal Chemistry 34, 1991, 1184-1187) describe the effect of sulfation in hirudin and hirudin PA related anticoagulant peptides as part of a structure activity relationship (SAR) study. Also, the synthesis of sulfamic acid derivatives of these peptides is given. R. Crosstick, *et al.* (Tetrahedron 40, 1984, 427-431) describe the synthesis of nucleoside analogues acting as antimetabolites (e.g. antiviral agents). One of the synthesised compounds is a sulfamic acid prepared from an amine and $SO_3$-triethylamine complex. I. Marle, *et al.* (Journal of Chromatography 586, 1991, 223-248) disclose a new chiral stationary phase for high-performance liquid chromatography based on cellulase. One of the solute structures is alfa-phenylethyl sulfamic acid. B.M. Kim, *et al.* (Tetrahedron letters 39, 1998, 5381-5384) describe new hydrolysis conditions for alfa-aminosulfamic acids, resulting in the formation of chiral diamines. These compounds are important building blocks in the synthesis of enzyme inhibitors. S.B. Cohen, *et al.* (Organic Letters 3, 2001, 405-407) present the synthesis of S-linked glycoconjugates via opening of a cyclic sulfamidate with 1-thio sugars and hydrolysis of the resulting sulfamic acids. J.E. Baldwin, *et al.* (Tetrahedron Asymmetry 1, 1990, 881-884) describe the nucleophilic opening of cyclic sulfamidates and subsequent hydrolysis of the in situ formed sulfamic acid is described.

When the sulfamic acid comprises, as R1 or R2, or both, an alkyl group, the alkyl group is preferably $C_1$-$C_{30}$ alkyl group; if the said alkyl group comprises a hetero atom, the said hetero atom is preferably selected from N, P, O and S.

When the sulfamic acid comprises, as R1 or R2, or both, an aromatic or heteroaromatic group, the said group preferably has 3-30 C-atoms; in case the said aromatic group is a heteroaromatic group, the hetero atom is preferably selected from N, P, O and S.

It is nevertheless observed that the above enumerations are not limitative; $R_1$ and $R_2$ can also represent other groups, provided that the sulfamic acid is still chiral.

Preferably, the sulfamic acid has an enantiomeric purity of at least 95%, more preferably of at least 99% or more. Most preferably, the sulfamic acid is homochiral. As outlined above, the resolution of the enantiomers in the mixture is dependent on the enantiomeric purity of the resolving agent; therefore, it is highly preferred for the sulfamic acid to be as enantiomerically (i.e. chirally) pure as possible.

As the salt formation of the sulfamic acid an the enantiomers in the mixture is an acid-base reaction, the enantiomers in the mixture to be resolved are preferably basic enantiomers. The enantiomers should at least be capable of being susceptible to an acid-base reaction with sulfamic acid to form the diastereomeric salts. The basic enantiomers are preferably amines.

According to the invention sulfamic acid can be used advantageously to resolve both racemic and non-racemic mixtures, e.g. wherein the molar ratio between both enantiomers is 1:10.

The invention further relates to a method for resolving a mixture of enantiomers B, comprising the steps of:

a) reacting in a liquid medium said mixture with a chiral sulfamic acid having an enantiomeric purity of at least 90% of formula (I):

$$(R_1, R_2)\ N\text{-}SO_3H \qquad (I)$$

as defined above, to obtain diastereomeric salts of formula (IV):

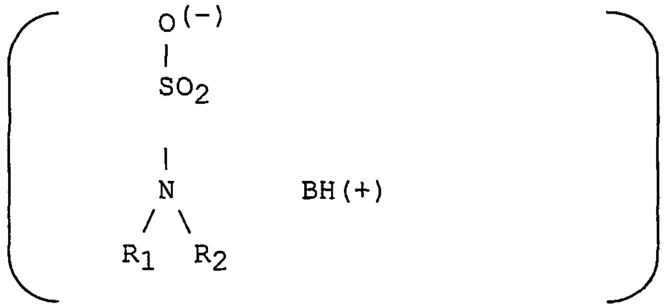

(IV)

resulting in preferential crystallization of either the p- or the n-diastereomeric salt,
b) recovering the crystallized salt obtained in step a),
c) treating the recovered crystallized salt of b) with a base to obtain the compound B in an enantiomerically enriched form, and, optionally,
d) recovering the sulfamic acid from c).

This process is schematically represented as follows:

Scheme 1:

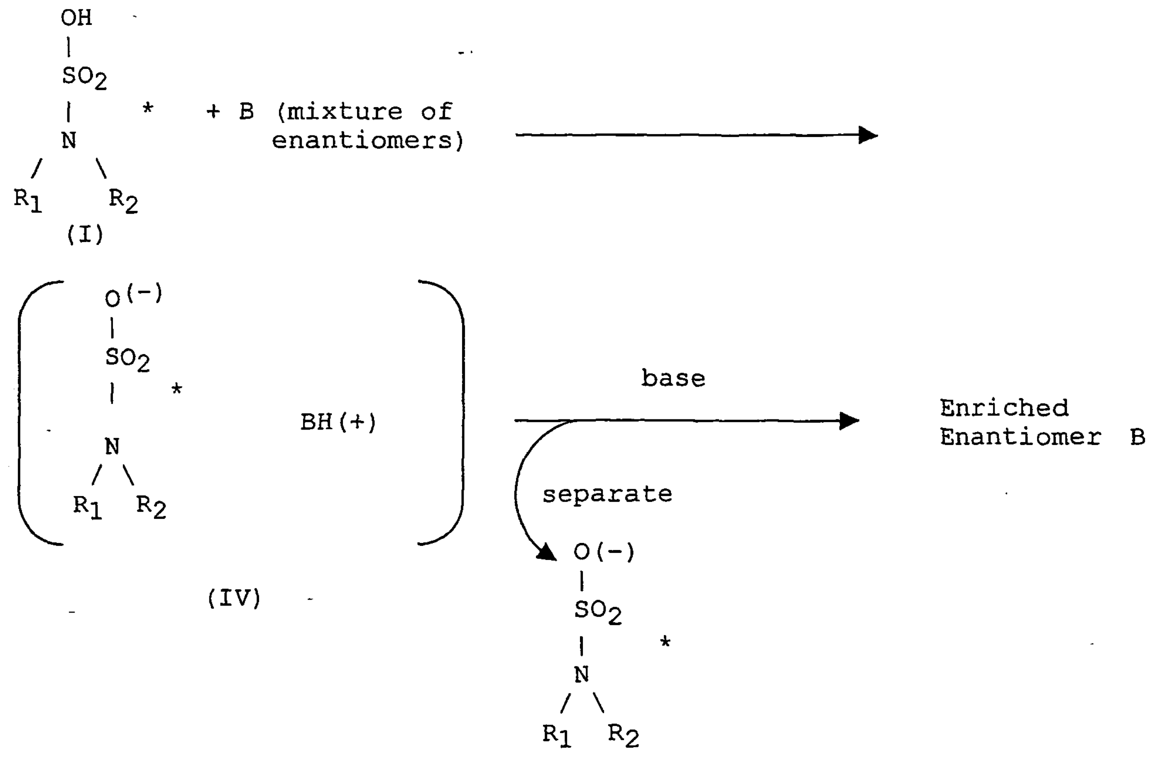


(I)

(IV)

(I)

According to the above method, enantiomeric mixtures can be resolved via intermediacy of sulfamate formation; in step a), the enantiomeric mixture is reacted with a sulfamic acid forming diastereomeric sulfamate salts through an acid-base reaction. Proper reaction conditions are known in the art; any skilled person will be aware of finding the conditions to conduct such a reaction. By this reaction, the p- or n- form of the diastereomeric salts crystallise and precipitate preferentially, as is outlined above, therewith leaving the remaining enantiomeric form, the antipode, in solution. The skilled person will be aware of suitable reaction conditions to obtain the envisaged preferential crystallisation.

Subsequently, the crystallised salt is recovered from the mixture by purification methods, known in the art. The recovered crystallised salt comprises the required enantiomer in enriched form. To obtain the enantiomer in the original form, the salt is treated with a base, so that the original enantiomer is released. It is to be understood that hereto, the said base should preferably be a stronger base than the enantiomer in the salt. Said enriched enantiomer can be further purified as known in the art. optionally, the sulfamic acid is recovered from the mixture by e.g. acidification of the mixture; an example of a suitable recovery is by ion exchange and subsequent freeze drying. The recovered sulfamic acid can be reused.

It is also possible to recover the envisaged enantiomer from the mother liquor, in case the salt of the said enantiomer remains in solution, and the salt of the antipode crystallises. In such a case, the mother liquor is enantiomerically enriched by removal of the precipitated crystals of the salt of the antipode. If both enantiomers are to be purified, both the salt crystals and the solubilised salt can be used for further purification of the respective enantiomers. Thus, in addition to or instead of steps b) and c) the diastereomeric salts from the liquid can be recovered and treated with a base to obtain compound B in an enantiomerically enriched form.

In order to obtain optimal resolution, as outlined above, the sulfamic acid has an enantiomeric purity of least 90%, preferably at least 95%, more preferably at least 99% or more and most preferably being homochiral. The best resolution is obtained when the sulfamic acid is as chirally pure as possible.

Preferably, as outlined above, the enantiomers B are basic enantiomers, preferably amines.

In a very special embodiment of the invention, the enantiomeric compounds of the mixture are amines, and the ($R_1$, $R_2$)N- moiety of the sulfamic acid used as resolving agent is stereochemically identical to the ($R_1$, $R_2$)N- moiety of one of the amines of the enantiomeric mixture. This is particularly advantageous when the n-salt of the sulfamic acid and the enantiomer crystallises. In that case, the crystallised salt constitutes of two chirally identical amine moieties that both can be purified; first, the n-salt is treated with a base as outlined above, resulting in release of the envisaged enantiomeric amine; second, the sulfamic acid can be recovered and treated, e.g. with an acid, to remove the $SO_3$ moiety therefrom resulting in stereochemically the same amine as released from the salt, therewith duplicating the yield. Thus, the purified product originates both from the enantiomeric mixture and from the resolving sulfamic acid. As outlined above, it is also possible to obtain the envisaged enantiomer in case the n-salt remains in solution, by recovering the said salt from the solution. Thereto, the mixture of basic enantiomers comprise an enantiomeric mixture of amines of formula (II):

$$(R_1, R_2)N\text{-}H \qquad (II),$$

wherein R1 and R2 are as above, and wherein the ($R_1$, $R_2$)N- moiety of the sulfamic acid of step a) is identical to at least one of the amines in the said enantiomeric mixture; in that case, "B" in the above scheme is

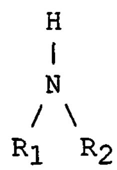

(II)

Very interestingly, it has also been found that the use of sulfamic acid as resolving agent enables the resolution of enantiomeric mixtures of amines of formula (II), as defined above, by first converting the said amines in an enantiomeric mixture of sulfamic acids, and by subsequent resolution of the thus formed enantiomeric sulfamic acid mixture with a suitable basic resolving agent; thereto the invention provides a method for resolving an enantiomeric mixture of amines of formula (II):

$$(R_1, R_2)N\text{-}H \qquad (II)$$

wherein $R_1$ and $R_2$ are as defined above, comprising the steps of:

1) reacting the said amines with a suitable reagent to obtain an enantiomeric mixture of corresponding sulfamic acid having formula (I) as defined above,
2) reacting the sulfamic acid of step 1) with a chiral basic resolving agent A having an enantiomeric purity of at least 90% to obtain diastereomeric salts of formula (V)

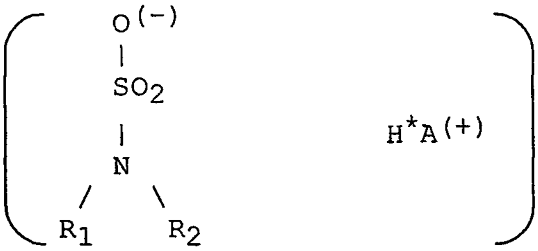

(V)

resulting in preferential crystallisation of either the p- or the n-diastereomeric salt,

3) recovering the crystallised salt obtained in step 2),

4) treating the recovered crystallised salt of step 3), with an acid to obtain the sulfamic acid of formula (I) in an enantiomerically enriched form,

5) recovering the amine of formula (II) from the sulfamic acid obtained in step 4) by removal of $SO_3$, and, optionally,

6) recycling the resolving agent A from step 4).

The above method is illustrated in the reaction scheme below:

Scheme 2:

step 1):

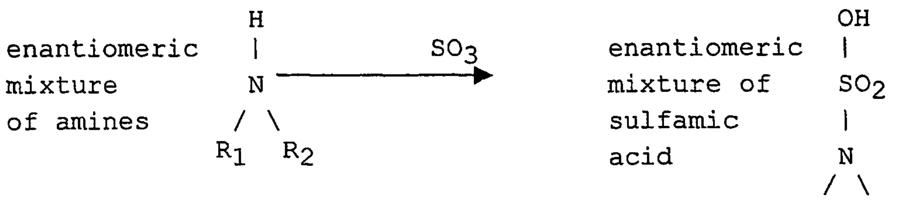


(II)

(I)

steps 2-3):

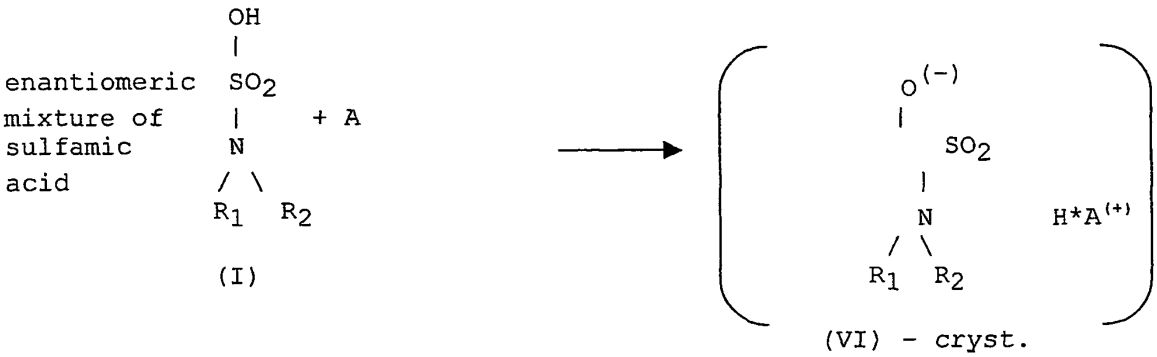


(I)

(VI) - cryst.

Step 4):

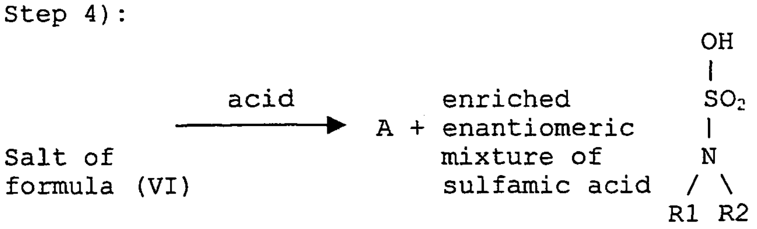


(I)

Step 5):

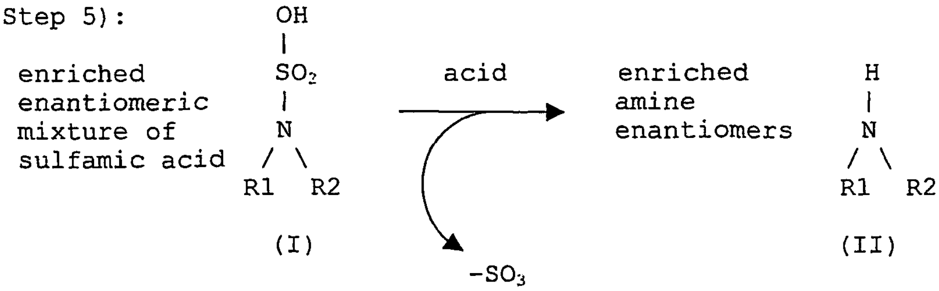


(I) (II)

Virtually all commercially available chiral amines can be used in the above process, for example,(R)-1-(4-nitrophenyl)ethylamine hydrochloride, (+)-dehydroabiethylamine, (S)-2-amino-1,1-diphenyl propanol, D-phenylalaninol, L(-)-α-amino-ε-caprolactam, (R)-(-)-1-amino-2-propanol, (R) -(+)-1-(1-naphthyl)ethylamine, (R)- (+) -1- (2-naphthyl) ethylamine, (R)-(+)-1-(4-bromophenyl)ethylamine, (R)-(+)-α-methylbenzylamine, (S)-(-)-1-(1-naphthyl)ethylamine, (S)-(-)-1-(2-naphthyl)ethylamine, (S) -(-) -1- (4-bromophenyl) ethylamine, (S) - (-)-α-methylbenzylamine, (S)-(+)-1-amino-2-propanol, (R)-(+)-bornylamine, (R)-(-)-phenylglycinol, (S)-(+)-phenylglycinol, D-(+)-phenylalaninol, L-(-)-phenylalaninol, (1S,2S)-(+)-2-amino-3-methoxy-1-phenyl-1-propanol, (-)-cis-myrtanylamine, D-(+)-norephedrine, L-(-)-norephedrine, (1R,2R)-(-)-2-amino-1-phenyl-1,3-propanediol, (1S,2S)-(+)-2-amino-1-phenyl-1,3-propanediol, (R)-(-)-1-aminoindan, (S)-(+)-1-aminoindan, (-)-isopinocamphenylamine, (+)-isopinocamphenylamine, (1R,2S)-(-)-cis-1-amino-2-indanol, (1S,2R)-(+)-cis-1-amino-2-indanol, (S)-2-phenylglycine methyl ester hydrochloride, (S)-2-phenylglycine methyl ester hydrochloride, (-)-L-phenylalanine benzyl ester, (R)-1-(3-methoxyphenyl)ethylamine, (S)-1-(3-methoxyphenyl)ethylamine, (R)-1- (4-methoxyphenyl) ethylamine, (S)-1-(4-methoxyphenyl)ethylamine, (1R,2S)-(+)-cis- [2- (benzylamino) cyclohexyl] methanol, (-)-bis [(S)-1-phenylethyl]amine hydrochloride, (-)-ephedrine, (+)-bis-[(R)-1-phenylethyl]amine hydrochloride, (+)-ephedrine hydrochloride, (1S,2R)-(-)-cis-[2-(benzylamino)cyclohexyl]methanol, (R)-benzyl-1-(1-naphthyl)ethylamine hydrochloride, (S)-1-(4-nitrophenyl)ethylamine hydrochloride, (S)-benzyl-1-(1-naphthyl)ethylamine hydrochloride, (R)-(+)-N-benzyl-1-phenylethylamine, (R)-(+)-N-methyl-1-phenylethylamine, (1R,2S)-(-)-N-methylephedrine, brucine, quinine, (-)-strychnine, cinchonidine, cichonine, quinidine, (R)-(+)-N,N-dimethyl-1-phenylethylamine, (S)-(-)-N,N-dimethyl-1-phenylethylamine, D-arginine, D-aspartic acid, D-glutamic acid, D-valine, L-aspartic acid, L-glutamic acid, L-valine.

Preferably, the suitable reagent to be used in the above process, is chlorosulfonic acid, sulfur trioxide, adducts of sulfur trioxide, such as in particular sulfur trioxide pyridine complex, sulfur trioxide dimethylformamide complex; also virtually any other adduct of sulfur trioxide can be used for the preparation of the envisaged sulfamic acids.

Because many starting amines are inexpensive reagents, and for the conversion thereof to sulfamic acids inexpensive reagents can be used, a basis for the large-scale use of sulfamic acids in diastereomer mediated resolutions is provided. The skilled person is aware of suitable reaction conditions for the preparation of the envisaged sulfamic acids.

Suitable basic resolving agents are known in the art, and can e.g. be chosen from the above-mentioned list of chiral amines.

In step 4), an acid should be used that is stronger than the sulfamic acid of the crystallised salt.

Treatment of a sulfamic acid to obtain the corresponding amine, the above step 5), are known in the art; preferably, step 5) comprises treatment of the sulfamic acid with chemical or thermal means, preferably by treatment with a hydrohalogenic acid, preferably hydrochloric acid. Steps 4) and 5) may preferably be combined by a single acid treatment. In addition to the above acids, the skilled person will be aware of suitable acids, such as e.g. sulfuric acid and nitric acid.

As outlined above, it is also possible to recover the envisaged enantiomer from the mother liquor, in case the salt of the said enantiomer remains in solution, and the salt of the antipode crystallises. In such a case, the mother liquor is enantiomerically enriched by removal of the precipitated crystals of the salt of the antipode. If both enantiomers are to be purified, both the salt crystals and the solubilised salt can be used for further purification of the respective enantiomers. Thus, in addition to or instead of steps 3) and 4) the diastereomeric salts from the liquid can be recovered and treated with an acid to obtain the sulfamic acid in an enantiomerically enriched form.

In a very special embodiment of the invention, the basic resolving agent is an amine, having a ($R_1$, $R_2$)N- moiety being stereochemically identical to the ($R_1$, $R_2$)N- moiety of one of the sulfamic acid enantiomers of the enantiomeric mixture. This is particularly advantageous when the n-salt of the sulfamic acid enantiomer and the resolving amine crystallises. In that case, the crystallised salt constitutes of two chirally identical amine moieties that both can be purified; first, the n-salt is treated with an acid as outlined above, resulting in release of the envisaged enantiomeric sulfamic acid; the amine is subsequently released from the said sulfamic acid by removal of the $SO_3$ moiety therefrom, e.g. with an acid. Second, the resolving amine, being stereochemically identical to that of the said sulfamic acid is recovered from the salt, resulting in duplication of the yield. Thus, the purified product originates both from the enantiomeric sulfamic acid mixture and from the resolving amine. As outlined above, it is also possible to obtain the envisaged enantiomer in case the n-salt remains in solution, by recovering the said salt from the solution. Thus, the basic resolving agent A preferably comprises an enantiomer of the amines to be resolved, which amines are first converted to the corresponding sulfamic acid.

According to the method according to the invention, it is also possible to resolve an enantiomeric mixture of sulfamic acids, without first converting an amine into the corresponding sulfamic acid; in that case, any enantiomeric mixture of sulfamic acids may be used and resolved (i.e. be enriched in one of the enantiomers of the mixture). Also, a further removal of $SO_3$ from the obtained enantiomerically enriched sulfamic acid is not necessary. Thus, the above steps 1) and 5) are not performed according to this embodiment of the invention.

As outlined above, it is highly preferred to use a resolving agent that is chirally as pure as possible, resulting in chirally highly enriched enantiomers, or even to chirally pure enantiomers (i.e. having a chiral purity of 95-100%, preferably 99-100%). Thereto, the resolving agent has an enantiomeric purity of at least 90%, preferably of at least 95%, more preferably of at least 99%; the resolving agent is most preferably homochiral.

Preferably, the molar ratio of the resolving agent:enantiomers in the mixture is substoichiometric and preferably 0.5 eq, as explained by Pope and Peachey in their method of half quantities, and disclosed in Enantiomers, Racemates and Resolutions, Wiley and Sons, New York (1981), pp. 309-313, herein incorporated by reference.

In an attractive embodiment of the invention, the enantiomeric mixture to be resolved is preferably a racemic mixture, in particular when substoichiometric amounts of resolving agent are used (see Pope and Peachy, supra).

The invention is now further illustrated by non-limitative examples.

Example 1

Preparation of D-phenylalaninol sulfamic acid

500 mg (3.31 mmol) D-phenylalaninol was dissolved in 10 ml of demi-water. The pH of the mixture was adjusted to pH 9.5-10.0 via addition of an aqueous 1.0 N NaOH solution. 659 mg (3.64 mmol)of sulfur trioxide pyridine complex was added in small portions over a period of 0.5 h with constant addition of 1.0 N NaOH solution to maintain a pH of 9.5-10.0. After the reaction was complete the reaction mixture was concentrated to remove the pyridine. Ethanol was added and the formed precipitate was removed by filtration and discarded. Acetone was added and the product precipitated from the solution. Filtration yielded 537 mg (2.32 mmol) of the sodium salt of D-phenylalaninol sulfamic acid (70 %). Next, 92 mg (0.36 mmol) of the sodium salt of D-phenylalaninol sulfamic acid was dissolved in 5 ml demineralized water. 3 ml of a suspension of Dowex-$H^+$ in demineralized water was added and stirred for 5 min. The mixture was filtered, the resin was rinsed 4 times with 5 ml of demineralized water and the collected aqueous filtrate was freeze-dried. 85 mg (0.36 mmol) of D-phenylalaninol sulfamic acid was obtained after freeze-drying.

Example 2

Resolution of racemic α-methylbenzylamine

80 mg (0.34 mmol) of D-phenylalaninol sulfamic acid was dissolved in 15 ml ethanol after which 43 mg (0.35 mmol) of racemic α-methylbenzylamine dissolved in 2 ml ethanol was added. The solvent was evaporated to a total volume of 3 ml and cooled down to accelerate crystallisation. After crystallisation both mother liquor and crystals were analysed by HPLC on a chiral ODH-column (eluent hexane: isopropylalcohol 90:10) to determine the relative amount of (S)- and (R)-α-methylbenzylamine. HPLC analysis showed enantiomeric enrichment of both the crystals and the

mother liquor.

Example 3

Preparation of (D)-1-(-3-methoxyphenyl)ethyl sulfamic acid and resolution of racemic α-methylbenzylamine

1.17 g (7.73 mmol) (D)-1-(-3-methoxyphenyl)ethyl amine was added dropwise to a suspension of 1.32 g (8.63 mmol) sulfur trioxide N,N-dimethylformamide complex in 15 ml of THF. After stirring for 10 min. TLC-analysis showed complete disappearance of primary amine (ninhydrine colouring). After evaporation of the solvents the sulfamic acid was obtained as a clear oil.
87 mg (0.38 mmol) of the sulfamic acid of (D)-1-(-3-methoxyphenyl)ethyl amine was dissolved in 1 ml of acetone and 48 mg (0.40 mmol) of racemic α-methylbenzylamine in 1 ml acetone was added. Within an hour crystals were formed and HPLC analysis showed enantiomeric enrichment of both the crystals and the mother liquor.

## Claims

**1.** Use of a chiral sulfamic acid having an enantiomeric purity of at least 90% of formula (I):

$$(R_1, R_2)N\text{-}SO_3H \qquad (I)$$

wherein:

$R_1$ and $R_2$, being the same or different, are selected from the group consisting of:

- hydrogen,
- a linear, branched or cyclic alkyl or heteroalkyl group, being optionally substituted, or
- an aromatic or heteroaromatic group, being optionally substituted, provided that $R_1$ and $R_2$ are not both hydrogen and that at least one of the $R_1$ and $R_2$ groups is chiral,

as resolving agent for resolving an enantiomeric mixture.

**2.** Use according to claim 1, wherein said alkyl group is a $C_1$-$C_{30}$ alkyl group, the hetero atom, if present, being selected from N, P, O and S.

**3.** Use according to claim 1 or 2, wherein said aromatic or heteroaromatic group has 3-30 C-atoms, the hetero atom, if present, being selected from N, P, O and S.

**4.** Method for resolving an enantiomeric mixture of enantiomers B, comprising the steps of:

a) reacting in a liquid medium said mixture with a chiral sulfamic acid having an enantiomeric purity of at least 90% of formula (I):

$$(R_1, R_2)N\text{-}SO_3H \qquad (I)$$

as defined in any of the claims 1-4, to obtain diastereomeric salts of formula (IV):

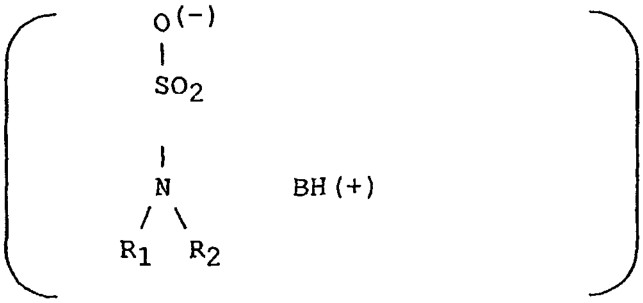

(IV)

resulting in preferential crystallization of either the p- or the n-diastereomeric salt,
b) recovering the crystallized salt obtained in step a),
c) treating the recovered crystallized salt of b) with a base to obtain the compound B in an enantiomerically enriched form, and, optionally,
d) recovering the sulfamic acid from c).

**5.** Method according to claim 4, wherein in addition to or instead of steps b) and c) the diastereomeric salts from the liquid are recovered and treated with a base to obtain compound B in an enantiomerically enriched form.

**6.** Method according to any of the preceeding claims, wherein an enantiomers B are basic enantiomers and wherein the mixture of enantiomers comprise an enantiomeric mixture of amines of formula (II):

$(R_1, R_2)N\text{-}H$ (II),

wherein R1 and R2 are as defined in any of the claims 1-4, and wherein the $(R_1, R_2)N$- moiety of the sulfamic acid of step a) is identical to at least one of the amines in the said enantiomeric mixture.

**7.** Method according to claim 6, further comprising step:

e) removing the $SO_3$ moiety of the sulfamic acid, recovered in step d) to obtain the amine of formula (II).

**8.** Method for resolving an enantiomeric mixture of amines of formula (II):

$(R_1, R_2)N\text{-}H$ (II)

wherein $R_1$ and $R_2$ are as defined in any of the claims 1-4, comprising the steps of:

1) reacting the said amines with a suitable reagent to obtain an enantiomeric mixture of corresponding sulfamic acid having formula (I) as defined in any of the claims 1-4,
2) reacting in a liquid medium the sulfamic acid of step 1) with a chiral basic resolving agent A having an enantiomeric purity of at least 90% to obtain diastereomeric salts of formula (v)

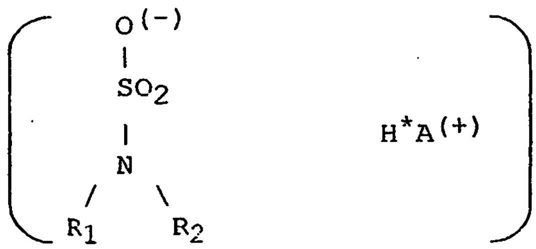

(V)

resulting in preferential crystallization of either the p- or the n-diastereomeric salt,
3) recovering the crystallised salt obtained in step 2),
4) treating the recovered crystallised salt of step 3), with an acid to obtain the sulfamic acid of formula (I) in an enantiomerically enriched form,
5) recovering the amine of formula (II) from the sulfamic acid obtained in step 4) by removal of $SO_3$, and, optionally,
6) recovering the resolving agent A from step 4).

**9.** Method according to claim 8, wherein in addition to or instead of steps 3) and 4) the diastereomeric salts from the liquid are recovered and treated with an acid to obtain the sulfamic acid in an enantiomerically enriched form.

**10.** Method according to claim 8 or 9, wherein the reagent of step 1) comprises chlorosulfonic acid, sulfur trioxide, adducts of sulfur trioxide, sulfur trioxide pyridine complex, sulfur trioxide dimethylformamide complex or a combination of two or more thereof.

**11.** Method according to any of the claims 7-10, wherein step e) and 5) respectively comprises treatment of the sulfamic

acid with chemical or thermal means, preferably by treatment with a hydrohalogenic acid.

**12.** Method according to any of the claims 8-11 wherein the basic resolving agent A comprises an enantiomer of the amines to be resolved.

**13.** Method for the resolution of an enantiomeric mixture of sulfamic acids having formula (I) as defined in claim 8, comprising steps 2), 3), 4) and optionally step 6) of claim 8.

**14.** Method according to any of the claims 4-13 wherein the molar ratio of resolving agent:entantiomers in the mixture to resolving agent is used in a substoichiometric amount and most preferably at 0.5 eq.

## Patentansprüche

**1.** Verwendung einer chiralen Amidosulfonsäure mit einer Enantiomerenreinheit von mindestens 90 % der Molekülformel (I):

$$(R_1, R_2)\ N - SO_3H \qquad (I)$$

wobei:

$R_1$ und $R_2$, die gleich oder unterschiedlich sind, ausgewählt werden aus der Gruppe, umfassend:

- Wasserstoff,
- eine lineare, verzweigte oder zyklische Alkyl- oder Heteroalkylgruppe, die wahlweise ersetzt ist, oder
- eine aromatische oder heteroaromatische Gruppe, die wahlweise ersetzt ist, vorausgesetzt, dass $R_1$ und $R_2$ nicht beide Wasserstoff sind und dass mindestens eine der $R_1$- und $R_2$-Gruppen chiral ist,

als Lösungsmittel zum Auflösen eines Enantiomerengemischs.

**2.** Verwendung nach Anspruch 1,
wobei die Alkylgruppe eine $C_1$-$C_{30}$-Alkylgruppe ist, wobei das Heteroatom, wenn vorhanden, aus N, P, O und S gewählt wird.

**3.** Verwendung nach Anspruch 1 oder 2, wobei die aromatische oder heteroaromatische Gruppe 3-30 C-Atome hat, wobei das Heteroatom, wenn vorhanden, aus N, P, O und S gewählt wird.

**4.** Verfahren zum Auflösen eines Enantiomerengemischs aus Enantiomeren B, umfassend die Schritte:

a) in einem Flüssigmedium das Gemisch mit einer chiralen Amidosulfonsäure mit einer Enantiomerenreinheit von mindestens 90 % der Molekülformel (I):

$$(R_1, R_2)\ N - SO_3H \qquad (I)$$

wie in einem der Ansprüche 1 bis 4 definiert, zur Reaktion bringen zum Erhalt von diastereomeren Salzen der Molekülformel (IV):

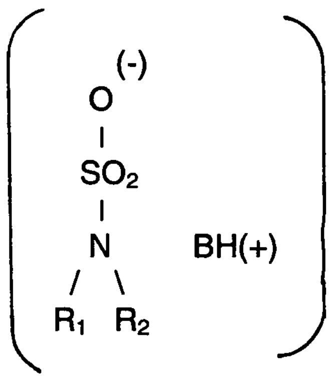

(IV)

mit dem Ergebnis der vorzugsweisen Kristallisation entweder des p- oder des n-diastereomeren Salzes,
b) Rückgewinnen des kristallisierten, in Schritt a) erhaltenen Salzes,
c) Behandeln des rückgewonnenen, kristallisierten Salzes aus b) mit einer Base zum Erhalt der Verbindung B in einer an einem Enantiomer angereicherten Form und, wahlweise,
d) Rückgewinnen der Amidosulfonsäure aus c).

**5.** Verfahren nach Anspruch 4,
wobei zusätzlich zu oder anstatt der Schritte b) und c) die diastereomeren Salze aus der Flüssigkeit rückgewonnen und mit einer Base zum Erhalt der Verbindung B in einer an einem Enantiomer angereicherten Form behandelt werden.

**6.** Verfahren nach einem der vorhergehenden Ansprüche,
wobei die Enantiomere B basische Enantiomere sind und ein Enantiomerengemisch von Aminen der Molekülformel (II):

$$(R_1, R_2)N\text{-}H \qquad (II),$$

umfassen und wobei $R_1$ und $R_2$ wie in den Ansprüchen 1 bis 4 definiert sind und wobei die $(R_1, R_2)N$-Komponente der Amidosulfonsäure aus Schritt a) identisch mit zumindest einem der Amine in dem Enantiomerengemisch ist.

**7.** Verfahren nach Anspruch 6, des weiteren umfassend den Schritt:

e) Entfernen der $SO_3$-Komponente der in Schritt d) zum Erhalt der Amine der Molekülformel (II) rückgewonnenen Amidosulfonsäure.

**8.** Verfahren zum Auflösen eines Enantiomerengemischs von Aminen der Molekülformel (II):

$$(R_1, R_2)N\text{-}H \qquad (II)$$

wobei $R_1$ und $R_2$ wie in einem der Ansprüche 1 bis 4 definiert sind, umfassend die Schritte:

1) die Amine mit einem geeigneten Reagens zur Reaktion bringen zum Erhalt eines Enantiomerengemischs der entsprechenden Amidosulfonsäure mit der Molekülformel (I) wie in einem der Ansprüche 1 bis 4 definiert,
2) in einem Flüssigmedium die Amidosulfonsäure aus Schritt 1) mit einem chiralen, basischen Lösungsmittel A mit einer Enantiomerenreinheit von mindestens 90% zum Erhalt von diastereomeren Salzen der Molekülformel (V)

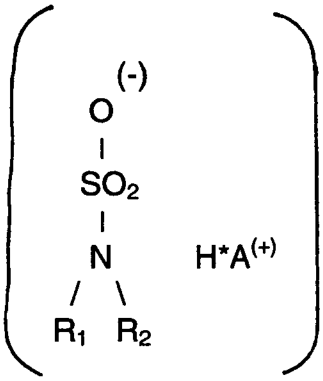

(V)

zur Reaktion bringen mit dem Ergebnis einer vorzugsweisen Kristallisation entweder des p- oder des n-diastereomeren Salzes,

3) Rückgewinnen des kristallisierten, in Schritt 2) erhaltenen Salzes,

4) Behandeln des rückgewonnenen, kristallisierten Salzes aus Schritt 3) mit einer Säure zum Erhalt der Amidosulfonsäure der Molekülformel (I) in einer an einem Enantiomer angereicherten Form,

5) Rückgewinnen des Amins der Molekülformel (11) aus der in Schritt 4) erhaltenen Amidosulfonsäure durch Entfernen von $SO_3$ und, wahlweise,

6) Rückgewinnen des Lösungsmittels A aus Schritt 4).

**9.** Verfahren nach Anspruch 8, wobei zusätzlich zu oder anstatt der Schritte 3) und 4) die diastereomeren Salze aus der Flüssigkeit rückgewonnen und mit einer Säure behandelt werden zum Erhalt der Amidosulfonsäure in einer an einem Enantiomer angereicherten Form.

**10.** Verfahren nach Anspruch 8 oder 9, wobei das Reagens von Schritt 1) Chlorschwefelsäure, Schwefeltrioxid, Schwefeltrioxidaddukte, einen Schwefeltrioxid-Pyridin-Komplex, einen Schwefeltrioxid-Dimethylformamid-Komplex oder eine Kombination von zweien oder mehreren derselben umfasst.

**11.** Verfahren nach einem der Ansprüche 7 bis 10, wobei Schritt e) bzw. 5) eine Behandlung der Amidosulfonsäure mit chemischen oder thermischen Mitteln, vorzugsweise eine Behandlung mit einer Halogenwasserstoffsäure, umfasst.

**12.** Verfahren nach einem der Ansprüche 8 bis 11, wobei das basische Lösungsmittel A ein Enantiomer der aufzulösenden Amine umfasst.

**13.** Verfahren zum Auflösen eines Enantiomerengemischs aus Amidosulfonsäuren mit der Molekülformel (I) wie in Anspruch 8 definiert, umfassend Schritte 2), 3), 4) und wahlweise Schritt 6) des Anspruchs 8.

**14.** Verfahren nach einem der Ansprüche 4 bis 13, wobei das Molverhältnis des Lösemittels: Enantiomeren in dem Gemisch zum Lösungsmittel in einer unterstöchiometrischen Menge verwendet wird und vorzugsweise mit 0,5 Äquivalenten.

**Revendications**

**1.** Utilisation d'un acide sulfamique chiral ayant une pureté énantiomère d'au moins 90 % de formule (I) :

$$(R_1, R_2)N\text{-}SO_3H \qquad (I)$$

dans laquelle :

$R_1$ et $R_2$, qui sont identiques ou différents, sont choisis dans le groupe consistant en :

- un hydrogène
- un groupe alkyle ou hétéroalkyle linéaire, ramifié ou cyclique, facultativement substitué, ou
- un groupe aromatique ou hétéroaromatique, facultativement substitué,

à condition que $R_1$ et $R_2$ ne soient pas tous les deux un hydrogène et à condition qu'au moins un des groupes $R_1$ et $R_2$ soit chiral,
comme agent de séparation pour séparer un mélange énantiomère.

**2.** Utilisation selon la revendication 1, dans laquelle ledit groupe alkyle est un groupe alkyle $C_1$-$C_{30}$, l'hétéroatome, s'il est présent, étant choisi parmi N, P, O et S.

**3.** Utilisation selon la revendication 1 ou 2, dans laquelle le groupe aromatique ou hétéroaromatique a 3 à 30 atomes de carbone, l'hétéroatome, s'il est présent, étant choisi parmi N, P, O et S.

**4.** Procédé pour séparer un mélange énantiomère d'énantiomères B, comprenant les étapes consistant à :

a) faire réagir dans un milieu liquide ledit mélange avec un acide sulfamique chiral ayant une pureté énantiomère d'au moins 90 % de formule (I) .

$(R_1, R_2)N\text{-}SO_3H$ (I)

comme défini dans l'une quelconque des revendications 1-4 pour obtenir des sels diastéréomères de formule (IV) :

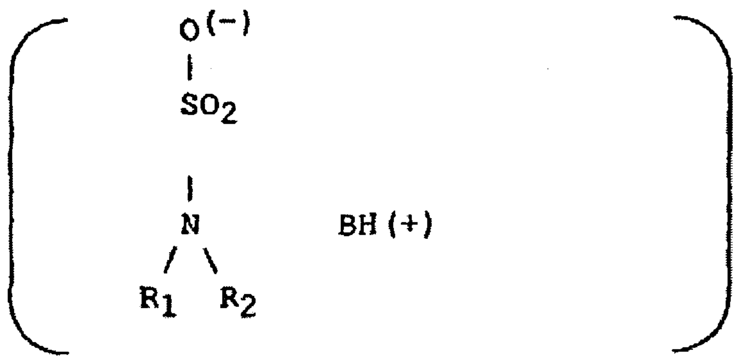

(IV)

entraînant la cristallisation préférentielle du sel diastéréomère p ou n,
b) récupérer le sel cristallisé obtenu à l'étape a),
c) traiter le sel cristallisé récupéré de b) avec une base pour obtenir le composé B sous une forme enrichie énantiomériquement et, facultativement,
d) récupérer l'acide sulfamique de c).

**5.** Procédé selon la revendication 4, dans lequel, en plus ou au lieu des étapes b) et c), les sels diastéréomères du liquide sont récupérés et traités avec une base pour obtenir le composé B sous une forme enrichie énantiomériquement.

**6.** Procédé selon l'une quelconque des revendications précédentes, dans lequel les énantiomères B sont des énantiomères basiques et dans lequel le mélange d'énantiomères comprend un mélange énantiomère d'amines de formule (II) :

**$(R_1, R_2)$ N-H (II)**

dans laquelle R1 et R2 sont comme défini dans l'une quelconque des revendications 1 à 4 et dans laquelle le groupement $(R_1, R_2)N$- de l'acide sulfamique de l'étape a) est identique à au moins une des amines dans ledit mélange énantiomère.

**7.** Procédé selon la revendication 6, comprenant en outre l'étape consistant à :

- éliminer le groupe $SO_3$ de l'acide sulfamique, récupéré à l'étape d) pour obtenir l'amine de formule (II).

**8.** Procédé pour séparer un mélange énantiomère d'amines de formule (II):

$$(R_1, R_2)N\text{-}H \qquad (II)$$

dans laquelle $R_1$ et $R_2$ sont comme défini dans l'une quelconque des revendications 1-4, comprenant les étapes consistant à :

1) faire réagir lesdites amines avec un réactif adapté pour obtenir un mélange énantiomère d'acide sulfamique correspondant ayant la formule (I) comme défini dans l'une quelconque des revendications 1 à 4,
2) faire réagir dans un milieu liquide l'acide sulfamique de l'étape 1) avec un agent de séparation basique chiral A ayant une pureté énantiomère d'au moins 90 % pour obtenir des sels diastéréomères de formule (V)

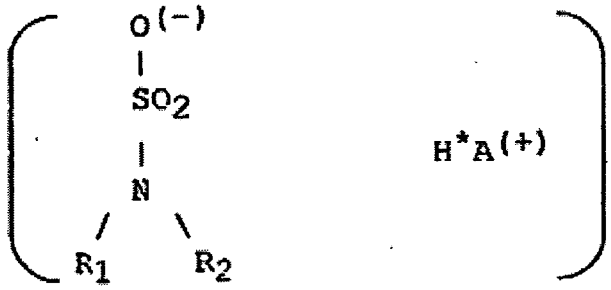

(V)

entraînant la cristallisation préférentielle du sel diastéréomère p ou n,
3) récupérer le sel cristallisé obtenu à l'étape 2),
4) traiter le sel cristallisé récupéré de l'étape 3) avec un acide pour obtenir l'acide sulfamique de formule (I) sous une forme enrichie énantiomériquement,
5) récupérer l'amine de formule (II) de l'acide sulfamique obtenu à l'étape 4) par élimination du $SO_3$, et, facultativement,
6) récupérer l'agent de séparation A de l'étape 4).

**9.** Procédé selon la revendication 8, dans lequel en plus ou au lieu des étapes 3) et 4) les sels diastéréomères provenant du liquide sont récupérés et traités avec un acide pour obtenir l'acide sulfamique sous une forme enrichie énantiomériquement.

**10.** Procédé selon la revendication 8 ou 9, dans lequel le réactif de l'étape 1) comprend de l'acide chlorosulfonique, du trioxyde de soufre, des adduits du trioxyde de soufre, un complexe trioxyde de soufre pyridine, un complexe trioxyde de soufre diméthylformamide ou une association de deux ou plus de ceux-ci.

**11.** Procédé selon l'une quelconque des revendications 7 à 10, dans lequel les étapes e) et 5) respectivement comprennent le traitement de l'acide sulfamique par des moyens chimiques ou thermiques, de préférence par traitement par un acide hydrohalogénique.

**12.** Procédé selon l'une quelconque des revendications 8 à 11, dans lequel l'agent de séparation basique A comprend un énantiomère des amines à séparer.

**13.** Procédé pour la séparation d'un mélange énantiomère d'acides sulfamiques ayant la formule (I) comme défini dans la revendication 8, comprenant les étapes 2), 3), 4) et facultativement l'étape 6) de la revendication 8.

**14.** Procédé selon l'une quelconque des revendications 4 à 13 dans lequel le ratio molaire agent de séparation : énantiomères dans le mélange d'agent de séparation est utilisé en une quantité sub-stoéchiométrique et de manière préférée entre toutes à 0,5 éq.